(19) Europäisches Patentamt
European Patent Office
Office européen des brevets

(11) Veröffentlichungsnummer : **0 167 207**
**B1**

(12) **EUROPÄISCHE PATENTSCHRIFT**

(45) Veröffentlichungstag der Patentschrift : **21.01.87**

(51) Int. Cl.⁴ : **C 07 C 43/313**, C 07 C 41/54

(21) Anmeldenummer : **85201007.3**

(22) Anmeldetag : **12.06.85**

(54) **Verfahren zur Herstellung von Keten-0,0-acetalen.**

(30) Priorität : **15.06.84 DE 3422217**

(43) Veröffentlichungstag der Anmeldung : **08.01.86 Patentblatt 86/02**

(45) Bekanntmachung des Hinweises auf die Patenterteilung : **21.01.87 Patentblatt 87/04**

(84) Benannte Vertragsstaaten : **BE CH DE FR GB LI**

(56) Entgegenhaltungen :
**EP-A- 0 108 322**
**DE-C- 566 033**
**RECUEIL DES TRAVAUX CHIMIQUES DES PAYS-BAS, Band 77, Nr. 11, November 1958, Seiten 1170-1188, D.B. Centen's Uitgeversmij., Hilversum, NL; H.C. VOLGER et al.: "Chemistry of acetylenic ethers XXXIV"**

(73) Patentinhaber : **BASF Aktiengesellschaft**
**Carl-Bosch-Strasse 38**
**D-6700 Ludwigshafen (DE)**

(72) Erfinder : **Ruland, Alfred, Dr.**
**Schriesheimer Strasse 11**
**D-6945 Hirschberg (DE)**
Erfinder : **Reuther, Wolfgang, Dr.**
**Am Pferchelhang 16**
**D-6900 Heidelberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein Verfahren zur Herstellung von Keten-O,O-acetalen durch Umsetzung von Phenoxyalkinen mit Phenolen. Sie können sehr leicht zu fungiziden Keten-O,N-acetalen weiterverarbeitet werden.

Zur Herstellung von Keten-O,O-acetalen sind mehrere Verfahren bekannt (D. Borrmann in Houben-Weyl-Müller, Methoden der organischen Chemie, Band 7/4, Seite 340 ff, Thieme Verlag, Stuttgart 1968) :

1. Abspaltung von Halogenwasserstoff aus alpha-Halogenacetalen mit Alkalialkoholaten, insbesondere Kalium-tert.-butanolat. Dieses Verfahren hat jedoch einige Nachteile. Zum einen sind damit in mäßigen bis mittleren Ausbeuten nur unsubstituierte oder einfach mit Chlor, Brom oder Phenyl substituierte Keten-O,O-acetale zugänglich, z. B. sinkt die Ausbeute beim Isopropyl-ketendiethylacetal bereits auf 22 % ab. Zum anderen handelt es sich bei den Alkoholkomponenten praktisch ausschließlich um einfache aliphatische Alkohole wie Methanol und Ethanol. Weiterhin sind die zur Synthese höher substituierter Keten-O,O-acetale mit Phenolen als Alkohol-Komponente benötigten alpha-Halogenacetale bisher nicht bekannt und aufgrund verschiedener Nebenreaktionen vermutlich nicht zugänglich.

2. Ein weiteres bekanntes Verfahren besteht in der Abspaltung von Alkoholen aus Orthocarbonsäuretriestern. Auch dieses Verfahren ist im Hinblick auf die Darstellung der benötigten Orthocarbonsäureester mit Phenolen als Alkoholkomponente praktisch nicht einsetzbar. Die gleiche Einschränkung gilt auch für die Synthese von Keten-O,O-acetalen durch Abspaltung von Alkylhypobromit aus alpha-Bromcarbonsäuretriestern.

3. Umsetzungen von 1,1-Dihaloethylenen zu Keten-O,O-acetalen sind ebenfalls bekannt. Sie gelingen aber nur bei beta-aktivierten Ethylenen oder im speziellen Fall des 1,1-Dichlorethylens durch Umsetzung mit beta- oder gamma-Alkoxy- bzw. Dialkylaminoalkoholaten.

4. Ein weiteres bekanntes Verfahren beschreibt die Herstellung von Diphenoxy-keten-O,O-acetalen aus den entsprechenden alpha-Hydroxyacetalen, durch Tosylierung der Hydroxylgruppe und nachfolgende Eliminierung. Dieses Verfahren liefert zwar gute Ausbeuten, ist aber aufgrund der nur mit relativ teuren Einsatzstoffen wie Natriumhydrid o. ä. durchführbaren Tosylierung für eine technische Herstellung unwirtschaftlich.

Der Erfindung lag die Aufgabe zugrunde, die Herstellung der Keten-O,O-acetale, die als Zwischenprodukte z. B. bei der Synthese von Fungiziden auf der Basis von Keten-O,N-acetalen interessieren, zu verbessern.

Es wurde nun gefunden, daß sich die genannten Nachteile der oben aufgezeigten Verfahren vermeiden lassen, wenn man Phenoxyalkine der Formel II

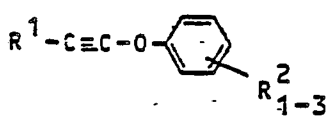

(II)

in der $R^1$ und $R^2$ die für Formel I genannte Bedeutung haben, mit Phenolen der Formel III

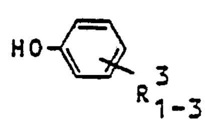

(III)

in der $R^3$ ebenfalls die für Formel I genannte Bedeutung hat, in Gegenwart von Friedel-Crafts-Katalysatoren umsetzt.

Alkylreste mit 1 bis 6 C-Atomen sind Methyl, Ethyl, n-Propyl, i-Propyl, n-, i- und tert.-Butyl, Pentyl, Hexyl. Halogen bedeutet hier Fluor, Brom, vorzugsweise Chlor.

Die Umsetzung der Phenoxyalkine der Formel II mit Phenolen der Formel III gemäß folgendem Reaktionsschema

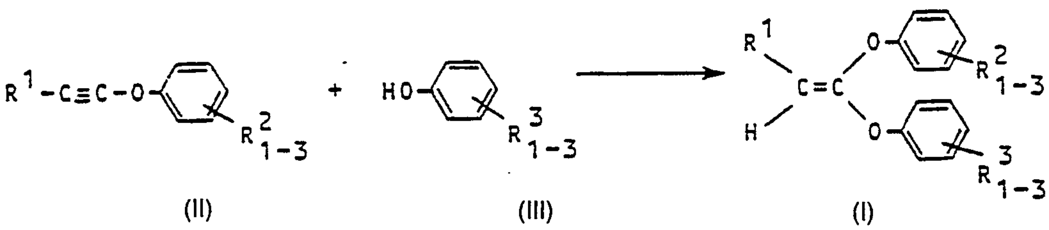

(II) (III) (I)

erfolgt in einem polaren, hochsiedenden, aprotischen Lösungsmittel wie Dimethylformamid, Dimethylsulfoxid, N-Methylpyrrolidon, Sulfolan, vorzugsweise in der Schmelze bei Temperaturen von 100 bis 200 °C, vorzugsweise bei 150 bis 170 °C in Gegenwart von Friedel-Crafts-Katalysatoren wie $AlCl_3$, $BF_3$, $SnCl_4$, $TiCl_4$, $CdCl_2$, vorzugsweise $ZnCl_2$. Als $R^2$ und $R^3$ wird Chlor bevorzugt, insbesondere, wenn sie mehrfach (zwei- oder dreifach) an jedem Phenylkern vorkommen. $R^2$ und $R^3$ können in 2-, 3- oder vorzugsweise 4-Stellung sitzen. Wenn sie zweifach pro Phenylkern vorkommen, sind die Positionen 2,4 und 3,5, bei dreifachem Vorkommen 2, 4, 5 bevorzugt. Es sind aber auch andere Positionenkombinationen möglich.

Das Reaktionsprodukt ist nur dann einheitlich, wenn die Phenylkerne im Phenoxyalkin II und im Phenol III in gleicher Weise substituiert sind. Andernfalls entstehen Gemische, deren Herstellung ebenfalls beansprucht wird. Im einfachsten Fall bestehen die Komponenten der Gemische nur aus den cis-trans-Isomeren. Je nach den Reaktionsbedingungen kann es aber auch in mehr oder weniger starkem Umfang zu einem Austausch der Phenylkerne kommen, so daß dann kompliziertere Gemische (aus 4 Komponenten) entstehen.

Umsetzungen von Alkoxyalkinen mit Alkoholen oder Phenolen sind im Prinzip zwar bekannt, z. B. aus V. Jäger und H. G. Viehe, Methoden der org. Chemie, Houben-Weyl-Müller, Thieme-Verlag Stuttgart 1977, Band 5/2a, S. 740, sie führen aber nur zu gemischt-aliphatisch-aromatischen Keten-O,O-acetalen und reagieren leicht zu den entsprechenden Orthoestern weiter. Darüberhinaus sind solche Umsetzungen auch nur mit unsubstituierten Alkoxyalkinen beschrieben. Umso überraschender ist daher die nahezu quantitative Umsetzung substituierter Phenoxyalkine mit Phenolen zu den entsprechenden Diphenoxyketen-O,O-acetalen.

Der große Vorzug dieser Verfahrensweise besteht darin, daß die benötigten Phenoxyalkine der Formel II leicht zugänglich sind (vgl. z. B. EP-A-118 082) und ihre Umsetzung mit Phenolen der Formel III gemäß vorliegender Erfindung nahezu quantitativ zu den gewünschten Verbindungen der Formel I erfolgt, so daß in der Regel für die weitere Umsetzung zu den fungiziden Keten-O,N-acetalen keine weitere Reinigung mehr erforderlich ist. Diese letztgenannte Umsetzung erfolgt gemäß EP-A-110 116 in der Schmelze bei ca. 180 bis 200 °C.

## Beispiel 1

1,1-Bis(2,4-dichlorphenoxy)-3,3-dimethylbut-1-en

24,3 g (0,1 Mol) 1-(2,4-Dichlorphenoxy)-3,3-dimethylbutin-1 wurden zusammen mit 16,3 g (0,1 Mol) 2,4-Dichlorphenol und 1,4 g (0,01 Mol) Zinkchlorid vorgelegt und unter Rühren auf 160 °C erhitzt. Nach 30 min hat sich das Phenol quantitativ mit dem Phenoxyalkin zum 1,1-Bis(2,4-dichlorphenoxy)-3,3-dimethylbut-1-en umgesetzt.

$^1$H-NMR-Daten (Trimethylsilan, in $CDCl_3$) :
$\delta$ = 1,2 (s, 9H) ; 4,95 (s, 1H) ; 7,73 (m, 6H)

## Beispiel 2

1,1-Bis(4-chlorphenoxy)-3,3-dimethylbut-1-en

31,3 g (0,15 Mol) 1-(4-Chlorphenoxy)-3,3-dimethylbutin-1 werden zusammen mit 19,3 g (0,15 Mol) 4-Chlorphenol und 1,4 g (0,01 Mol) Zinn-IV-chlorid vorgelegt und unter Rühren auf 160 °C erhitzt, bis mittels Hochdruckflüssigkeitschromatographie kein Phenoxyalkin mehr nachweisbar ist. Dies ist nach 20 bis 60 min der Fall.

$^1$H-NMR : $\delta$ = 1,2 (s, 9H) ; 4,8 (s, 1H) ; 6,8-7,4 (m, 8H)

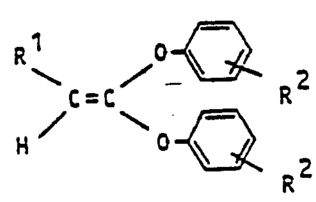

In analoger Weise wurden die Verbindungen der Beispiele 3 bis 7 hergestellt :

| Beispiel | $R^1$ | $R^2$,$R^3$ | $^1$H-NMR-Daten $\delta$-Werte in $CDCl_3$ |
|---|---|---|---|
| 3 | 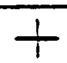 | 2-Cl | $\delta$= 1,2 (s,9H); 4,8 (s,1H); 6,8-7,4 (m,8H) |

Tabelle (Fortsetzung)

| Bei-spiel | $R^1$ | $R^2$,$R^3$ | $^1H$-NMR-Daten δ-Werte in $CDCl_3$ |
|---|---|---|---|
| 4 | 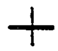 | 4-Br | δ = 1,15 (s,9H); 4,8 (s,1H); 6,8-7,1 (m,6H) |
| 5 | 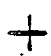 | 3,5-$Cl_2$ | δ = 1,15 (s,9H); 4,95 (s,1H); 6,8-7,1 (m,6H) |
| 6 | 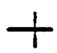 | 2,4,5-$Cl_3$ | δ = 1,2 (s,9H); 4,95 (s,1H); 7,25-7,5 (m, 4H) |
| 7 | 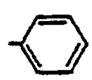 | $R^2$=2,4-$Cl_2$ $R^3$=4-Cl | δ = 5,8 (s,1H); 7,1-7,8 (m,12H); 5,9 (s,1H); 7,1-7,8 (m,12H); E/Z-Isomerengemisch |

**Patentanspruch**

Verfahren zur Herstellung eines Keten-O,O-acetals der Formel I

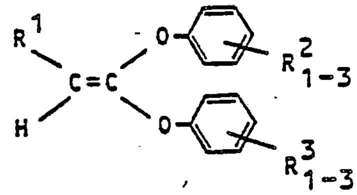

(I)

in der $R^1$ einen Alkylrest mit 1 bis 6 C-Atomen oder einen gegebenenfalls durch Halogen oder Phenyl substituierten Phenylrest und $R^2$ und $R^3$ unabhängig voneinander Chlor, Brom oder einen Phenylrest bedeuten, dadurch gekennzeichnet, daß man ein Alkin der Formel II

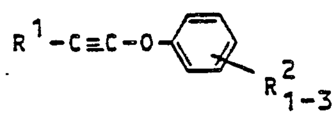

(II)

in der $R^1$ und $R^2$ die für Formel I genannte Bedeutung haben, mit einem Phenol der Formel III

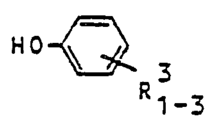

(III)

in der $R^3$ ebenfalls die für Formel I genannte Bedeutung hat, in Gegenwart von Friedel-Crafts-Katalysatoren umsetzt.

**Claim**

A process for the preparation of a ketene O,O-acetal of the formula I

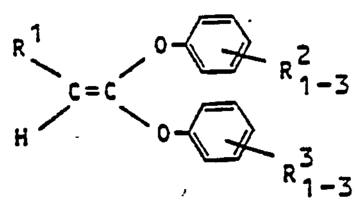

(I)

where $R^1$ is alkyl of 1 to 6 carbon atoms or phenyl which is unsubstituted or substituted by halogen or phenyl, and $R^2$ and $R^3$ independently of one another are chlorine, bromine or phenyl, wherein an alkyne of the formula II

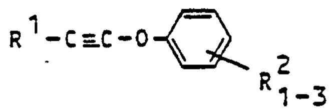

(II)

where $R^1$ and $R^2$ have the meanings given for formula I, is reacted with a phenol of the formula III

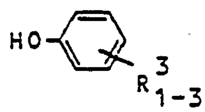

(III)

where $R^3$ likewise has the meanings given for formula I, in the presence of a Friedel-Crafts catalyst.

**Revendication**

Procédé de préparation d'O,O-acétals de cétènes de la formule I

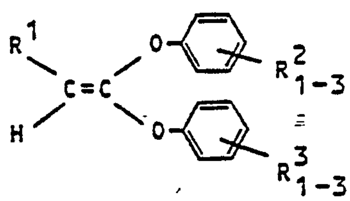

(I)

dans laquelle $R^1$ désigne un radical alkyle en $C_1$ à $C_6$ ou un groupe phényle pouvant être halo- ou phényl-substitué et $R^2$ et $R^3$ désignent indépendamment l'un de l'autre du chlore ou du brome ou un groupe phényle, caractérisé en ce que l'on fait réagir une alcyne de la formule II

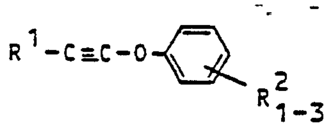

(II)

dans laquelle $R^1$ et $R^2$ possèdent la signification définie pour la formule I, en présence d'un catalyseur de Friedel-Crafts, avec un phénol de la formule III

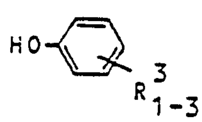

(III)

dans laquelle $R^3$ possède la signification définie pour la formule I.